(19) Europäisches Patentamt
European Patent Office
Office européen des brevets

(11) **EP 3 689 225 A1**

(12) **EUROPÄISCHE PATENTANMELDUNG**

(43) Veröffentlichungstag:
**05.08.2020  Patentblatt 2020/32**

(51) Int Cl.:
***A61B 3/00*** (2006.01)          ***A61B 3/103*** (2006.01)
***A61B 3/14*** (2006.01)

(21) Anmeldenummer: **20154521.7**

(22) Anmeldetag: **30.01.2020**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **30.01.2019  DE 102019102373**

(71) Anmelder: **Fielmann Ventures GmbH
22083 Hamburg (DE)**

(72) Erfinder: **SCHROETER, Franziska
20257 Hamburg (DE)**

(74) Vertreter: **Seemann & Partner Patentanwälte mbB
Raboisen 6
20095 Hamburg (DE)**

(54) **VERFAHREN, SOFTWAREPRODUKT UND SYSTEM ZUR REFRAKTIONSBESTIMMUNG AUF EINEM MOBILEN ENDGERÄT**

(57)    Die Erfindung betrifft ein Verfahren und ein Softwareprodukt zur Refraktionsbestimmung auf einem mobilen Endgerät (20), sowie ein System.

Das mobile Endgerät wird in einem Aufnahmeabstand vor einem der Refraktionsbestimmung zu unterziehenden Messsubjekt (10) positioniert, dessen Augen (12) einer exzentrischen Photorefraktion zu unterziehen sind, so dass die Kamera (24, 26) und die Lichtquelle (28) auf das Gesicht des Messsubjekts (10) gerichtet sind. Es werden Testbilder des Messsubjekts (10) ohne und/oder mit zugeschaltetem Licht aus der Lichtquelle (28) aufgenommen und Aufnahmeabstands A, Aufnahmeparameter, Lichtbedingungen und/oder Ausrichtung des mobilen Endgeräts (20) zur Person (10) anhand der Testbilder optimiert, bis eine zur exzentrischen Photorefraktion geeignete Einstellung gefunden ist. Ferner wird eine Mehrzahl von Messaufnahmen des Messsubjekts (10) aufgenommen, die Mehrzahl von Messaufnahmen im Zuge einer Refraktionsauswertung ausgewertet, wobei Werte für die winkelabhängige Brechkraft eines oder beider Augen (12) ermittelt werden, insbesondere Werte für *Sph*, *Cyl* und *Achse.* Die Messergebnisse werden ausgegeben und/oder gespeichert.

Erfindungsgemäß wird oder werden bei der Auswertung der Testbilder, der Messaufnahmen und/oder bei der Refraktionsauswertung Machine Learning, insbesondere neuronale Netze, eingesetzt.

Fig. 1

EP 3 689 225 A1

**Beschreibung**

Die Erfindung betrifft ein Verfahren zur Refraktionsbestimmung auf einem mobilen Endgerät sowie ein Softwareprogramm zur Ausführung des erfindungsgemäßen Verfahrens.

Korrektionsbrillen können bestimmte Fehlsichtigkeiten eines Menschen ausgleichen, wenn die optische Brechkraft beider Augen unter Berücksichtigung der Sehstärke (Visus) korrekt bestimmt werden. Zu den Fehlsichtigkeiten gehören beispielsweise die Ametropie, die Heterophorie oder die Presbyopie. Die Brechkraftbestimmung geschieht in der Regel bei einem Augenarzt oder Augenoptiker bei der sogenannten Refraktionsbestimmung oder Augenglasbestimmung mit speziellen kalibrierten Messgeräten. Häufig gliedert sich diese in eine objektive (Vor-)Refraktion mithilfe zum Beispiel eines Autorefraktometers, und eine subjektive Refraktion mittels einer Messbrille oder eines Phoropters. Bei den genannten Messinstrumenten handelt es sich zumeist um große, teure Geräte, die in der Regel in einer bestimmten Anordnung zur Sehprobentafel und bei genormten Lichtverhältnissen in speziellen Refraktionsräumen aufgestellt werden müssen.

Bei der subjektiven Refraktion muss der Nutzer aktiv Rückmeldung zu bestimmten Seheindrücken geben, was bei der objektiven Refraktion nicht der Fall ist. Die objektive Refraktion ist daher häufig deutlich schneller als die subjektive Refraktion. Jedoch ist der subjektive Seheindruck des Nutzers ausschlaggebend für die Verträglichkeit der Brille und somit der Nutzerzufriedenheit, so dass die objektive Refraktion häufig als Ausgangspunkt für die subjektive Refraktion verwendet wird.

Grundsätzlich ist es sinnvoll, eine objektive Refraktion vor der subjektiven Refraktion vorzunehmen, um für die anschließende langwierigere subjektive Refraktion geeignete Ausgangsparameter zu erhalten und mit einem bereits akzeptablen Ausgangswert beginnen zu können.

Bislang erfolgt die objektive Refraktion üblicherweise mittels eines teuren und immobilen Geräts in einem Optikfachgeschäft oder beim Augenarzt, welches nur für eine einzelne Messung gleichzeitig verfügbar ist. Eine Messung außerhalb der Geschäftsräume eines Augenoptikers oder der Praxisräume eines Augenarztes wird bislang nicht praktiziert.

Es existieren einige Ansätze, eine Photorefraktion auf andere Art und Weise als bisher zu bewerkstelligen. So offenbart US 9,380,938 B2 ein System und ein Verfahren zur Dokumentation, Aufnahme und Interpretation exzentrischer Photorefraktion, pupillärer Lichtreflexe und von Lichtreflexen auf der Hornhaut bei Kleinkindern. Das System umfasst ein mobiles Endgerät mit einer Kamera und einer Lichtquelle, wobei Fotos aufgenommen und zu einem Zentralcomputer hochgeladen werden, der sie verarbeitet und auswertet. Dem Bediener werden graphische Hinweise zur Positionierung der Aufnahmen gegeben. Die Auswertung umfasst eine Auswertung der Position und Größe der Möndchen in den Pupillen, die bei Fehlsichtigkeit durch die exzentrische Anordnung der Lichtquelle gegenüber der Kamera entstehen.

Auch US 9,877,649 B2 offenbart ein System und ein Verfahren zur Durchführung einer photorefraktiven Untersuchung mithilfe eines mobilen Endgeräts bei Kindern, ohne direkt die Brechkraft zu messen. Die technische Lehre von US 9,877,649 B2 konzentriert sich auf die Einhaltung geeigneter Rahmenbedingungen für die Aufnahmen, die jeweils auf ihre Verwertbarkeit hinsichtlich Positionierung, Helligkeit, oder Aufnahmewinkel hin überprüft werden. Eine Bedienperson wird gegebenenfalls angewiesen, Maßnahmen zu ergreifen, um in den folgenden Aufnahmen die akzeptablen Aufnahmeparameter einzuhalten.

In DE 10 2016 112 023 A1 wird eine Komponente für eine mobile Computervorrichtung, beispielsweise ein Smartphone, bereitgestellt, welche an einem Gehäuse der mobilen Computervorrichtung angebracht werden kann. Die Komponente kann mittels eines Optikelements das Licht einer eingebauten Lichtquelle der mobilen Computervorrichtung umlenken und gegebenenfalls filtern, oder kann eine eigene Lichtquelle bereitstellen, um die Möglichkeit, eine exzentrische Photorefraktionsmessung mit der mobilen Computervorrichtung durchzuführen, zu verbessern. Somit wird durch die Komponente das System kompliziert und für die Heimanwendung untauglich.

Es ist daher die Aufgabe der vorliegenden Erfindung, die Refraktionsbestimmung flexibler als bisher zugänglich zu machen.

Diese Aufgabe wird durch ein Verfahren zur Refraktionsbestimmung an einem Messsubjekt, dessen Augen einer exzentrischen Photorefraktion zu unterziehen sind, auf einem mobilen Endgerät gelöst, welches eine Kamera mit einer Linse und eine schaltbare Lichtquelle umfasst, die mit einem festen Abstand neben der Linse angeordnet ist, mit den folgenden Verfahrensschritten:

a) Positionieren des mobilen Endgeräts in einem initialen Aufnahmeabstand vor dem Messsubjekt, so dass die Kamera und die Lichtquelle auf das Gesicht des Messsubjekts gerichtet sind,

b) Aufnehmen von Testbildern des Messsubjekts ohne und/oder mit zugeschaltetem Licht aus der Lichtquelle und Optimieren des Aufnahmeabstands in Abhängigkeit von der Brechkraft des Auges oder der Augen des Messsubjekts sowie Optimieren von Aufnahmeparametern, von Lichtbedingungen und/oder der Ausrichtung des mobilen Endgeräts zum Messsubjekt anhand der Testbilder, bis eine zur exzentrischen Photorefraktion geeignete Einstellung gefunden ist,

c) Aufnehmen einer Mehrzahl von Messaufnahmen des Messsubjekts, wobei das mobile Endgerät durch Rotation um eine Achse, die parallel zur optischen Achse der Linse verläuft, nacheinander in zwei oder mehr Winkelorientierungen gebracht wird und in jeder Winkelorientierung eine oder mehrere Messaufnahmen aufgenommen werden,

d) Auswerten der Mehrzahl von Messaufnahmen im Zuge einer Refraktionsauswertung, wobei Werte für die winkelabhängige Brechkraft eines oder beider Augen ermittelt werden, insbesondere Werte für *Sph, Cyl* und/oder Achse,

e) Ausgeben und/oder Speichern der Messergebnisse, insbesondere der Werte für *Sph, Cyl* und/oder Achse eines oder beider Augen des Messsubjekts,

wobei bei der Auswertung der Testbilder, der Messaufnahmen und/oder bei der Refraktionsauswertung Machine Learning, insbesondere neuronale Netze, in dem mobilen Endgerät oder in einem mit dem mobilen Endgerät datenverbundenen Server eingesetzt wird oder werden.

Die erhöhte Flexibilität zeigt sich beispielsweise in der erhöhten Mobilität der Refraktionsbestimmung, die nunmehr auch von Zuhause aus erfolgen kann oder außerhalb des Refraktionsraums innerhalb eines Augenoptikfachgeschäft.

Eine Grundidee der vorliegenden Erfindung ist es, eine exzentrische Photorefraktion mit einem mobilen Endgerät durchzuführen und diese durch den Einsatz von Machine Learning und gegebenenfalls neuronalen Netzen zu unterstützen. Ein mobiles Endgerät ist im Rahmen der vorliegenden Anmeldung vorzugsweise ein Smartphone, ein Handy oder ein Tablet, welches, zumeist auf der Rückseite, mit einer Kombination aus einer oder mehrerer Kameras und einem Photolicht oder Blitzlicht ausgestattet ist. Da die Kamera und das Blitzlicht fest in dem Gerät verbaut sind, ist ihre geometrische Beziehung zueinander, d. h. ihre Orientierung zueinander und ihr Abstand voneinander, festgelegt und bekannt. Die Exzentrizität der Aufnahmeanordnung für die exzentrische Photorefraktion ist somit bekannt. Neben den erwähnten Geräten sind beispielsweise auch Laptops als mobile Endgeräte im Sinne der Erfindung umfasst, sofern sie mit Blitzgeräten oder Fotolichtern ausgestattet sind.

Die exzentrische Photorefraktion ist ein bekanntes Verfahren, welches in vielen zu diesem Zweck konstruierten Geräten verwendet wird, beispielsweise Autorefraktometern oder Screeninggeräten für Kinder, die bei Augenärzten oder in Optikfachgeschäften zum Einsatz kommen.

Hauptkomponenten der exzentrischen Photorefraktion sind eine Lichtquelle (häufig ein Blitzlicht) und eine Kamera, deren Abstand Exzentrizität genannt wird. In der Regel ist die Verbindungslinie zwischen der Lichtquelle und der Kamera im Wesentlichen senkrecht zur optischen Achse des Auges, welches untersucht wird. Wenn das Auge normalsichtig ist, dann nehmen nach dem Prinzip der geometrischen Optik die Strahlen von der Lichtquelle ins Auge den gleichen Weg auch wieder zurück, d.h. die Kamera erkennt keinen Widerschein der Lichtquelle, da das von der Lichtquelle ausgesandte Licht zur Lichtquelle zurückkehrt. Wenn das Auge jedoch fehlsichtig ist, dann erreicht ein Teil der reflektierten Strahlen die Kamera und es ist ein sogenanntes *Möndchen* (Englisch: Crescent) in der Pupille auf den Kamerabildern zu sehen, d.h. ein heller Lichtreflex, der in der Regel kontinuierlich in den dunkleren, unbelichteten Teil der Pupille übergeht. Anhand der Größe und der Position, aber auch anhand der Intensitätsverteilung dieses Möndchens lässt sich auf den optischen Brechwert des Auges schließen.

Eine in T.D. Cole, "Multimeridian photorefraction: A Technique for the detection of visual defects in infants and preverbal children", Johns Hopkins APL Technical Digest, Vol. 12, No. 2 (1991) (im Folgenden Referenz [1]) hergeleitete Formel beschreibt den Zusammenhang zwischen Möndchengröße und der Brechkraft des Auges. Wenn e die Exzentrizität in Metern ist, d der Abstand zwischen Kamera und Nutzer in Metern, r der Radius der Pupille in Metern, s die Möndchengröße in Metern und A die Brechkraft des Auges Dioptrien sind, dann gilt:

$$A = \frac{1}{d}\left(\frac{e}{2r-s} + 1\right).$$

Neben der korrekten Bestimmung der Möndchengröße s müssen somit auch der Abstand d zwischen Kamera und Nutzer sowie die Exzentrizität e und der Pupillenradius r genau ausgemessen werden oder bekannt sein. Der Abstand zwischen Nutzer und Kamera kann z.B. über die Pupillendistanz ermittelt werden, falls diese beispielsweise aus einer zuvor vorgenommenen Vermessung oder aus früheren Messdaten bekannt ist, oder z.B. über mittels einer gegebenenfalls im mobilen Endgerät verbauten Tiefenkamera. Die Verwendung bekannter früherer Messergebnisse kann die Genauigkeit und Verlässlichkeit der Resultate verbessern. Weitere Sensoren des mobilen Endgeräts, die bei der Auswertung der Messaufnahmen verwendet werden können, sind beispielsweise Beleuchtungssensoren, Beschleunigungssensoren, Tiefenkameras oder Abstandssensoren, soweit vorhanden.

Bei der Bestimmung der Pupillendistanz kann auch ein hardware- und/oder softwaregestütztes Eye-Tracking und/oder Landmark-Tracking zum Einsatz kommen. Mittels des Eye-Tracking kann unter anderem sichergestellt werden, dass das Messsubjekt eine geeignete Blickrichtung einnimmt.

Ferner gibt es aus augenoptischer Sicht in der Regel nicht "die" eine Brechkraft des Auges. Diese Beschreibung trifft nur dann zu, wenn die Augenlinse nur eine sphärische Verkrümmung aufweist, also in jeder Richtung die gleiche Verkrümmung bzw. Dioptrienzahl. Dies bedeutet, dass die Gesamtheit der optischen Elemente des Auges (Hornhaut, Augenlinse, Linsenflüssigkeit) sich durch eine sphärische Linse approximieren lassen. Tatsächlich muss allerdings häufig die Gesamtheit der optischen Elemente des Auges als Torus angenähert werden. In dem Fall liegt eine Hornhautverkrümmung (Astigmatismus) vor. Dies bedeutet, dass die Fehlsichtigkeit bzw. Dioptrienzahl in einer Richtung (beispielsweise horizontal) anders ist als in einer dazu orthogonalen Richtung (beispielsweise vertikal). Die Hauptachsen der Fehlsichtigkeit, bei denen die maximale und minimale Fehlsichtigkeit bzw. Dioptrienzahl erreicht werden, müssen aber nicht horizontal und vertikal liegen, sondern können unter beliebigen Winkeln zur Vertikalen und Horizontalen liegen. Sie müssen auch nicht notwendigerweise orthogonal aufeinander sein. Allerdings liegen die Hauptachsen üblicherweise ungefähr orthogonal zueinander, so dass eine Annahme der Orthogonalität der Hauptachsen eine gute Annäherung an die realen Verhältnisse darstellt, zumal Brillengläser in der Regel mit zueinander orthogonalen Hauptachsen gefertigt werden.

Unter der Annahme der Orthogonalität der Hauptachsen der Fehlsichtigkeit wird die Fehlsichtigkeit üblicherweise mit drei Werten, die im Brillenpass gebräuchlich sind, beschrieben. Diese Werte sind der *sphärische Wert (Sph),* der *zylindrische Wert (Cyl)* und die *Achse (A).*

Diese Werte erklären sich anhand der sogenannten Hauptschnittwirkungen. Als Hauptschnittwirkung wird die Brechkraft zugehörig zu dem jeweiligen Hauptschnitt (entsprechend den jeweiligen Hauptachsen) bezeichnet, die sowohl positiv als auch negativ sein kann. Es existieren verschiedene Konventionen. In der sogenannten Minus-Zylinder-Schreibweise stimmt der sphärische Wert *Sph* mit der mathematisch größeren Hauptschnittwirkung überein, also der positiveren der beiden Dioptrienzahlen, und der zylindrische Wert entspricht einem Minuszylinder (plankonkav) mit dem Wert der astigmatischen Differenz. Die Achse wird zwischen der Waagerechte und dem Hauptschnitt zugehörig zum sphärischen Wert gemessen.

Bei der Auswertung ist darauf zu achten, dass alle drei Werte sorgfältig bestimmt werden. In der Regel erfolgt dies über die Messung der Möndchen unter verschiedenen Winkeln des mobilen Endgeräts zum Nutzer, d.h. es wird auf multimeridionale Photorefraktion zurückgegriffen, wie sie z.B. in dem bereits genannten Artikel (Referenz [1]) beschrieben ist. Dazu wird das Endgerät um eine Achse gedreht, die senkrecht zu der Verbindungslinie zwischen Kamera und Blitzlicht verläuft, so dass die Verbindungslinie zwischen Kamera und Blitzlicht in Bezug auf den Nutzer gedreht wird. Die einfachsten Fälle sind die Aufstellung oder Halterung des mobilen Endgeräts im Hochformat und im Querformat, also vertikal und horizontal, jedoch können bei geeigneter Halterung oder Abstützung auch Winkel dazwischen eingenommen werden.

Hierbei ist es wichtig, den Winkel möglichst genau zu bestimmen. Dies erfolgt beispielsweise durch die Bestimmung der Positionen der Pupillen des Nutzers in den aufgenommenen Bildern bzw. Messaufnahmen, wobei die Verbindungslinie zwischen den Pupillen eine Referenzorientierung für die Orientierung des mobilen Endgeräts in Bezug auf den Nutzer darstellt. Es können auch Daten aus der inertialen Messeinheit (*"inertial measurement unit", IMU,* also Beschleunigungssensor, Gyroskop und/oder Magnetoskop) des mobilen Endgeräts benutzt werden, um dessen Neigung bzw. Orientierung zu bestimmen.

In diesem Zusammenhang wird im Verfahrensschritt c) das mobile Endgerät zum Aufnehmen einer Mehrzahl von Messaufnahmen des Messsubjekts durch Rotation um eine Achse, die parallel zur optischen Achse der Linse verläuft, nacheinander in zwei oder mehr Winkelorientierungen gebracht, und es werden in jeder Winkelorientierung eine oder mehrere Messaufnahmen aufgenommen. Die Minimalanzahl von zwei Winkelorientierungen kann dann zur Bestimmung von *Sph* und *Cyl* verwendet werden, wenn der Wert Achse bereits bekannt ist, beispielsweise aus Vormessungen. Ansonsten handelt es sich um drei Werte, für deren Messung mindestens drei verschiedene Winkelorientierungen verwendet werden müssen, beispielsweise 0°, 45° und 90°, alternativ 0°, 60° und 120°, oder 0°, 120° und 240°.

Alternativen zur rein geometrischen Auswertung der Möndchen stellen die Auswertung des Helligkeitsverlaufes des Möndchens dar, z.B. nach A. Roorda, M. C. W. Campbell: "Geometrical theory to predict eccentric photorefraction intensity profiles in the human eye", Journal ofthe Optical Society of America A, Vol. 12, No. 8 (1995) (im Folgenden Referenz [2]), und der Ray-Tracing-Ansatz für astigmatische Augen, z.B. nach W. Wesemann, A. M. Norcia, D. Allen: "Theory of eccentric photorefraction (photoretinoscopy): astigmatic eyes", Journal of the Optical Society of America A, Vol. 8, No. 12 (1991) (im Folgenden Referenz [3]).

Praktisch werden für die multimeridionale Photorefraktion analog zu Referenz [1] in mehreren verschiedenen Meridianen bzw. Winkellagen des mobilen Endgeräts zwischen 0° und 180° Bilder aufgenommen, bzw. auch zwischen 180° und 360°, und aus diesen die jeweilige Möndchengröße bestimmt bzw. eine Bildauswertung bezüglich der Möndchen, etwa des Helligkeitsverlaufs, ein Raytracing oder eine anderweitige Analyse durchgeführt. Daraus lässt sich in der Folge der zusammengesetzte Sehfehler in den jeweiligen Meridianen bestimmen. Aus den einzelnen Messwerten lassen sich durch einen Fit einer Fitfunktion für die über den Meridianwinkel aufgetragenen Messwerte die Werte für Sphäre, Zylinder und Achse in einer gewählten Notation bestimmen.

Bei Vorhandensein einer Referenzdatenbank mit Fotos unterschiedlicher Benutzer für unterschiedliche Meridiane bei jeweils bekannten Werten für Sphäre, Zylinder und Achse lassen sich auch neuronale Netzwerke trainieren, die auf der Grundlage der für das Messsubjekt im Rahmen des erfindungsgemäßen Verfahrens aufgenommenen Fotos aus dem Erscheinungsbild des jeweils aufgenommenen Möndchens in den verschiedenen Fotos der verschiedenen Meridiane die Werte für Sphäre, Zylinder und Achse ermitteln. Bei dem Training sind für die als Referenz dienenden unterschiedlichen Benutzer nicht nur die jeweiligen Werte für Sphäre, Zylinder und Achse bekannt, sondern auch die weiteren Aufnahmeparameter wie Abstand und Exzentrizität der jeweiligen Aufnahmen, sowie gegebenenfalls weitere Parameter wie die Pupillendistanz des jeweiligen Benutzers. Das Ergebnis ist nicht eine analytische Auswertung der genauen Form der Möndchen, sondern ein auf die direkte Bewertung der Aufnahmen trainiertes neuronales Netz, welches bei der Analyse eines neuen Satzes von Aufnahmen mit ebenfalls bekannten Werten für Aufnahmeabstand und Exzentrizität sowie gegebenenfalls der Pupillendistanz des Benutzers unter anderem direkt zum Ergebnis für die Werte für Sphäre, Zylinder und Achse gelangt. Alternativ kann ein solches neuronales Netz auch auf der Grundlage von Simulationsdaten trainiert werden.

Eine im Rahmen der Erfindung verwendbare Alternative zur multimeridionalen exzentrischen Refraktionsbestimmung stellt die sogenannte bimeridionale Auswertung dar, bei der Messaufnahmen lediglich in zwei zueinander senkrecht stehenden Winkelorientierungen aufgenommen werden, wie beispielsweise in dem bereits zitierten Artikel von Wesemann et al. (Referenz [3]) beschrieben wurde. Aus diesen wird die Größe des schwarzen Anteils der Pupille und dessen Rotation gegen die Horizontale gemessen. Aus dem theoretischen Zusammenhang, wonach diese Werte paarweise durch ein Polardiagramm mit den Werten Sphäre, Zylinder und Achse verbunden sind, lassen sich dann die gemessenen Werte auf die gesuchten Brillenpasswerte umrechnen.

In dieser Alternative werden in einer Ausführungsform der Erfindung für eine bimeridionale Auswertung Messaufnahmen in zwei zueinander senkrecht stehenden Winkelorientierungen aufgenommen, insbesondere im Hoch- und Querformat. Alternativ oder zusätzlich dazu werden in Ausführungsformen für eine multimeridionale Auswertung Messaufnahmen in wenigstens drei, vorzugsweise vier, fünf oder mehr, verschiedenen Winkelorientierungen aufgenommen, insbesondere in Winkelabständen von 30°. Es können auch beliebige andere Abstände gewählt werden, sofern für die multimeridionale Auswertung genügend unterschiedliche und nicht redundante Winkelpositionen gewählt werden.

In Ausführungsformen wird an der Mehrzahl von Testbildern und/oder Messaufnahmen eine bimeridionale oder multimeridionale Refraktionsauswertung ausgeführt, wobei an der Mehrzahl von Testbildern und/oder Messaufnahmen jeweils eine Bildanalyse durchgeführt wird, wobei in den Messaufnahmen das Vorhandensein, Größe, Orientierung, Ort, Form und/oder Intensitätsverlauf von reflektiertem Licht, welches in den Messaufnahmen als Möndchen in den Pupillen des Messsubjekts erscheint, sowie der Aufnahmeabstand A ermittelt werden. Diese Bildanalyse kann entweder direkt nach der Aufnahme und noch vor der darauf folgenden Aufnahme ausgeführt werden, oder im Nachgang, nachdem mehrere oder alle der Messaufnahmen vollendet wurden. Die unmittelbare Auswertung erlaubt es, bei minderwertigen Aufnahmen diese zu wiederholen, bis eine Aufnahme gemacht ist, die zu einem sicheren Ergebnis führt. Bei den Testbildern, die unter Verwendung des Blitzlichts des mobilen Endgeräts aufgenommen wurden, wird auf das Vorhandensein des Möndchens abgezielt. Gegebenenfalls wird noch die Vorbereitung dadurch geführt, dass die Möndchen beispielsweise im Wesentlichen die Hälfte der Pupille ausführen. Bei stark unterschiedlicher Fehlsichtigkeit zwischen dem linken und dem rechten Auge können auch zwei Testreihen und Messreihen für das linke und rechte Auge jeweils durchgeführt werden, anderenfalls wird vorzugsweise eine Mittelposition, in der in beiden Augen Möndchen erscheinen, gewählt.

Vorzugsweise wird ein Aufnahmeabstand gewählt, der etwas, insbesondere bis zu 40%, insbesondere bis zu 20%, größer als der Fernpunkt wenigstens eines Auges des Messsubjekts ist. Dies bedeutet, dass das Messsubjekt das mobile Endgerät als unscharf wahrnimmt. Ansonsten würde die Fokussierung durch das Auge das Entstehen eines Möndchens verhindern. Eine mögliche Vorgehensweise hierzu ist, einen Abstand zu wählen, in dem das Möndchen etwa die Hälfte der Pupille ausfüllt. Der Fernpunkt ist ein Parameter der Brechkraft des Messsubjekts und kann entweder bereits vorbekannt sein, oder auf einfache Weise ermittelt werden. Bei der Verwendung eines mobilen Endgeräts kommen zur Bestimmung des Fernpunkts zum Beispiel die folgenden Überlegungen zum Zuge. Der Messbereich des Verfahrens liegt bei aktuellen mobilen Endgeräten aufgrund der toten Zone (Englisch: "dead zone"), also des Dioptrienbereichs, in dem verfahrensbedingt mit Photorefraktion keine Messung möglich ist, im Bereich zwischen ca. -0.75 dpt und ca. -5 dpt, was einem Fernpunkt bei ca. 4/3 m = 1,33 m und ca. 1/5 m = 20 cm Entfernung entspricht. Das Messsubjekt kann sich also beispielsweise zur Bestimmung des Fernpunkts in einer Entfernung von 1,5 m positionieren und sukzessive Testbilder aufnehmen, wobei das Messsubjekt in Schritten näher an das mobile Endgerät herantritt, bis es in ca. 20 cm Entfernung vom mobilen Endgerät steht. Die jeweilige konkrete Distanz des Messsubjekts zum Endgerät ergibt sich beispielsweise aus den Testbildern selbst, wenn die Pupillendistanz bekannt ist. Wenn auf allen Testbildern keine Möndchen zu sehen sind, dann liegen entweder die Refraktionswerte des Messsubjekts außerhalb des Messbereichs des verwendeten mobilen Endgeräts oder das Verfahren hat aus sonstigen Gründen nicht funktioniert. In diesem Fall kann beispielsweise in einem Augenoptik-Fachgeschäft eine Refraktionsbestimmung durchgeführt werden.

Um einen möglichst effizienten Ablauf der Messung sicherzustellen, wird in einer Ausführungsform im Verfahrensschritt b) oder c) zu jeder Messaufnahme geprüft, ob diese verwertbar ist, und eine Änderung der Winkelorientierung des Endgeräts dann veranlasst, wenn in der bisherigen Winkelorientierung eine festgelegte oder ausreichende Anzahl verwertbarer Messaufnahmen aufgenommen worden ist, wobei insbesondere die Bildanalyse einer Messaufnahme unmittelbar nach dem Aufnehmen der Messaufnahme im Verfahrensschritt c) erfolgt.

Vorzugsweise wird oder werden in den Messaufnahmen das Gesicht des Messsubjekts, die Augen, die Iris und/oder die Pupille(n) erkannt und ihre Größe, insbesondere in Pixeln, bestimmt, sowie wird oder werden das oder die Möndchen innerhalb der erkannten Iris oder Irides (Plural von Iris) erkannt. Dies kann deterministisch erfolgen, beispielsweise durch Nutzung von digitalen Filtern, beispielsweise eines Kantenfilter, linearer und nichtlinearer Regression oder ähnlichem. Alternativ werden in einer Ausführungsform der Erfindung bei der Erkennung des Gesichts, der Augen, der Iris, der Pupille und/oder des Möndchens ein oder mehrere neuronale Netzwerke eingesetzt, das oder die zuvor für die entsprechende Erkennung trainiert worden war oder waren. Ein Beispiel für neuronale Netzwerke, die bei der Gesichtserkennung und auch bei der Erkennung anderer Gegenstände in Bildern häufig und erfolgreich eingesetzt werden, sind die sogenannten neuronalen Faltungsnetzwerke ("convolutional neural networks", CNN), welche anhand von Bibliotheken von Beispielsbildern trainiert werden und auf diese Weise die Erkennung und Klassifizierung von Gegenständen oder Gesichtern in Fotos oder anderen Aufnahmen mit hoher Sicherheit ausüben.

Entsprechende neuronale Netzwerke können trainiert werden, Gesichter zu erkennen, im Gesicht das Auge oder die Augen zu erkennen und im Auge die Iris und gegebenenfalls auch ein Möndchen zu erkennen. Dabei wird die Größe der Pupille und möglicherweise des Möndchens in Pixeln bestimmt. Es kann auch die Lage und/oder der Helligkeitsverlauf des Möndchens in der Pupille bestimmt werden. Nachfolgend können dann ausgehend von einem oder mehreren Fotos mit Möndchen und der Messung von weiteren Größen wie dem Abstand zwischen Nutzer und Kamera Messwerte für Sphäre, Zylinder und Achse ermittelt werden.

Die Gesichtserkennung mithilfe neuronaler Netze ist bekannt. Hilfreich dafür sind die Neural Engines in Apples iPhones ab dem Modell iPhone X® und iPhone Xs® sowie entsprechende Chips anderer Hersteller. Neben oder anstelle der Neural Engines oder vergleichbarer Chips können auch die üblicherweise vorhandenen Grafikprozessoreinheiten ("GPU") verwendet werden, die die maschinelle Bildverarbeitung erheblich beschleunigen und gegebenenfalls für Machine Learning verwendet werden können. Diese werden z. B. in Apples iPhone X® und iPhone Xs® und Nachfolgemodellen verbaut. Es existieren sowohl proprietäre als auch Open Source Frameworks und Methoden, die beim Machine Learning verwendet werden. Hierzu gehört auch im Rahmen der vorliegenden Erfindung die Möglichkeit des Einsatzes von Frameworks, die es ermöglichen, Gesichter wiederzuerkennen (Face Recognition), wenn zum Beispiel ein Selfie mit der Frontkamera des mobilen Endgeräts aufgenommen, das Gesicht samt Landmarks gespeichert wird und dann bei der Verwendung der Rück-Kamera wiedererkannt wird. Damit wird beispielsweise sichergestellt, dass für die richtige Person die Brechkraft der Augen bestimmt wird.

Eine zweite Anwendung ist der Einsatz von neuronalen Netzen, um die Iris, die Pupille und das Möndchen im Auge zu erkennen. Dabei kann mit deterministischen Methoden nach Kreis- oder Ellipsenformen im Bild des Auges aufgrund von lokalen Helligkeitsunterschieden im Auge gesucht werden, wobei in der Regel die Iris rund und grau, grün, blau, braun, schwarz oder Mischungen davon ist. Jedoch kann es aufgrund der eher schlechten Auflösung der Bilder des mobilen Endgeräts zu Bildern der Iris kommen, bei denen der Rand nicht ganz rund ist und daher die Suche nach Kreis- und Ellipsenformen zu Fehlergebnissen führt. Die Suche nach Kreis- oder Ellipsenformen wird weniger fehleranfällig, wenn Netze aus dem Bereich Semi-supervised Learning wie *generative adversarial networks* (GAN) eingesetzt werden oder Netze aus dem Bereich Supervised Learning wie z.B. *convolutional neural networks* (CNN). Es können hier auch gemischte neuronale Netze verwendet werden (zum Beispiel kombinierte GAN/CNN). Es können auch vortrainierte Netze verwendet werden, um z.B. Resultate von einem mobilen Endgerät auf ein anderes übertragen zu können, die auch auf dem mobilen Endgerät selbst (fertig) trainiert werden können, z.B. auf der Neural Engine des iPhone Xs® oder dem iPad Pro®.

Im Weiteren können neuronale Netze eingesetzt werden, um die Größe von Pupille und Möndchen in Pixeln und die Lage oder den Helligkeitsverlauf des Möndchens in der Pupille zu bestimmen. Hierbei werden die vorhandenen analytischen Methoden durch eigens trainierte neuronale Netze verbessert, weil Bilder von Möndchen im Gegensatz zu Bildern von Pupillen nicht in gängigen Datenbanken vorhanden sind und die Möndchen selten uniform oder wohlgeformt sind. Außerdem gibt es meistens keine klare Grenzlinie zwischen dem Möndchen und der Pupille, so dass die Grenzlinie erst einmal gefunden werden muss. Aufgrund der eher schlechten Auflösung der Bilder, im Rahmen der Erfindung bedingt durch den Einsatz von mobilen Endgeräten, sowie gegebenenfalls der Aufnahme von Bildern in größerem Abstand zum Nutzer, sind das Möndchen und Pupille häufig nur wenige Pixel groß. Für die Genauigkeit der Ergebnisse der Brechkraft des Auges ist es entscheidend, die Größe des Möndchens und der Pupille so genau wie möglich zu bestimmen. Die Grenzlinie zwischen Möndchen und Pupille kann auch in der Mitte zwischen zwei Pixeln verlaufen.

Gemäß einer Ausführungsform wird die Messung noch verbessert, indem bei der Bestimmung von Größe, Lage und/oder Helligkeitsverlauf des Möndchens oder der Möndchen eine durch Machine Learning unterstützte Superresolution verwendet wird, die insbesondere unter Verwendung von neuronalen Netzen durchgeführt wird, die anhand von Bildern in hoher und niedriger Auflösung trainiert worden sind, insbesondere dahingehend, die Kante eines Möndchens in der Pupille zu erkennen und zu verdeutlichen. Superresolution bedeutet zunächst, dass ein Bild mit geringer Auflösung durch sogenanntes Up-Sampling zu einem Bild mit höherer Auflösung umgerechnet wird. So kann im einfachsten Fall jedes Pixel beispielsweise in vier Subpixel aufgeteilt werden, denen jeweils anhand des Pixels und der umgebenden Pixeln interpolierte Helligkeitswerte zugewiesen werden, beispielsweise mittels bilinearer oder bikubischer Interpolation. Im Unterschied dazu wird eine durch Machine Learning unterstützte Superresolution beispielsweise unter Verwendung von neuronalen Netzen durchgeführt, die anhand von Bildern in hoher und niedriger Auflösung trainiert worden sind. Dies kann das Funktionieren der Analyse sowohl in klassischer Bildauswertung als auch durch neuronale Netzwerke verbessern, da die Pupillen im Auge des Nutzers in den Messaufnahmen einen Durchmesser von nur wenigen, gegebenenfalls weniger als 10, Pixeln aufweisen. Eine Superresolution kann dies besser auswertbar machen, insbesondere, wenn das erzeugende neuronale Netz oder GAN darauf trainiert worden ist, die Kante eines Möndchens in der Pupille zu erkennen und zu verdeutlichen. Dies kann dann als Input für die Bildanalyse, aber auch für neuronale Netze eingesetzt werden, insbesondere solche, die sich hierfür bewährt haben. Dies liefert sinnvolle Ergebnisse, wenn dafür neuronale Netze auf entsprechenden Bildern trainiert werden.

In einer Ausführungsform wird ein System künstlicher Intelligenz, insbesondere ein neuronales Netzwerk oder eine andere Ausführungsform von Machine Learning, eingesetzt, um ausgehend von einem oder mehreren Messaufnahmen mit Möndchen und den zugehörigen Messparametern Messabstand und Exzentrizität der Lichtquelle Messwerte für *Sph, Cyl* und *Achse* zu ermitteln, wobei das System künstlicher Intelligenz zuvor mit Messaufnahmen mit Möndchen von Referenzsubjekten mit bekannten Werten von *Sph, Cyl* und *Achse* trainiert worden war.

Dies ist möglich, wenn ein Datensatz mit einer Vielzahl von Menschen vorliegt, bei denen sowohl Fotos bzw. Messaufnahmen der Möndchen für eine bimeridionale oder eine multimeridionale Photorefraktion als auch die schon bekannten Werte für Sphäre, Zylinder und Achse vorliegen. Ein neuronales Netz kann dann daraufhin trainiert werden, die Ermittlung der drei Werte aus den Messaufnahmen mit großer Sicherheit und Genauigkeit nachzuvollziehen. Ein weiterer Vorteil dabei besteht darin, dass auch die Verschiedenheit der Erscheinungsbilder der Menschen, beispielsweise verschiedene Augenfarben, Augenformen etc. mittrainiert wird. Dies stellt sicher, dass das Ergebnis der Auswertung nicht abhängig ist von dem Erscheinungsbild des individuellen Nutzers.

Neben der oben bereits beschriebenen multimeridionalen Auswertung und der oben erwähnten bimeridionalen Auswertung nach Wesemann (Referenz [3]) kann unter Einsatz von neuronalen Netzen auch eine Auswertung der Verteilung der Lichtintensität im Möndchen erfolgen. So können in mindestens zwei Bildern bzw. Messaufnahmen Ellipsen an die Bereiche konstanter relativer Helligkeit geplottet werden. Die Mittelpunkte dieser Ellipsen sind abhängig von Sphäre, Zylinder und Achse und ermöglichen somit ein Zurückrechnen auf diese Werte. Hierzu sind Bilder lediglich in zwei Orientierungen notwendig, es handelt sich somit um eine alternative bimeridionale Auswertung.

Die Ellipsen können bezüglich der Lichtintensitätsverteilung im Möndchen über neuronale Netze analysiert werden. Auch hierfür können neuronale Netze mit Referenzfotos und bekannten Werten trainiert werden. Die Verwendung der Helligkeitsinformationen des Möndchens hat gegenüber der rein geometrischen Analyse der Kantendetektion den Vorteil, dass die Menge der verfügbaren Informationen in Form der Helligkeitswerte der einzelnen Pixel oder gegebenenfalls Subpixel wesentlich größer ist als die in den rein geometrischen Begrenzungen enthaltenen Informationen. Daraus kann eine höhere Genauigkeit resultieren. Das Prinzip der Auswertung wurde zum Beispiel in dem Artikel Kusel et al., "Light-intensity distribution in eccentric photorefraction crescents", J. Opt. Soc. Am. A, Vol. 15, No. 6, 1998 (Referenz [4]), beschrieben.

Eine weitere Alternative der Analyse von exzentrischer Photorefraktion beruht auf der Auswertung des Gradienten der Helligkeitsverteilung in der Pupille entlang dem betrachteten Meridian. Hierbei werden auch Linsenfehler beachtet. Über einen Zusammenhang zwischen dem Gradienten ("slope") der Helligkeitsverteilung, dem Sehfehler, insbesondere dem sphärischen Äquivalent, und Abstand ist eine Auswertung über den Gradienten möglich. Die Methode wurde im Grundsatz beschrieben in Roorda et al., "Geometrical theory to predict eccentric photorefraction intensity profiles in the human eye", J. Opt. Soc. Am. A/Vol. 12, No. 8, 1995 (Referenz [5]).

Eine weitere Beschreibung einer Auswertung auf der Grundlage von neuronalen Netzen ist beispielsweise in Costa et al., "Photorefraction images analysis through neural networks", Optics, 2010 (Referenz [6]) beschrieben worden. Darin wurden zwei neuronale Netze eingerichtet, welche eine Reihe von Pixeln der gemachten Bilder in Verbindung mit der Pupillengröße (Fläche), sowie der Pupillendistanz als Input-Datensatz erhielten. In der vorhergehenden Bildbearbeitung wird der Hornhautreflex detektiert und herausgerechnet/markiert. Der Unterschied zwischen den beiden neuronalen Netzen ist, dass sie auf unterschiedliche Genauigkeiten ausgelegt wurden und somit mit unterschiedlich viel Input, als auch unterschiedlich vielen Neuronen ausgestattet wurden. Die Verwendung neuronaler Netze bietet bei der Auswertung der Möndchen den Vorteil, dass keine genaue, deterministische Bildauswertung des Möndchens nötig ist. Es wurde in Referenz [6] gezeigt, dass eine Genauigkeit von 0,25 dpt für Werte zwischen -3 bis -1 dpt erreicht werden konnte.

Es können verschiedene Algorithmen eingesetzt werden, um die Güte und Verlässlichkeit der Messergebnisse zu beurteilen. Auch hierbei können sowohl deterministisch Methoden wie die klassische Statistik eingesetzt werden, als auch Methoden aus dem Bereich der künstlichen Intelligenz, insbesondere aus dem Machine Learning. In einer Ausführungsform wird oder werden daher Güte und/oder Verlässlichkeit der Messergebnisse berechnet und bei nicht ausreichender Güte und/oder Verlässlichkeit wird das Messsubjekt aufgefordert, einzelne oder alle Schritte der Messung zu wiederholen oder eine objektive Refraktionsbestimmung beim Augenoptiker oder Augenarzt durchführen zu lassen.

In einer Ausführungsform wird zusätzlich eine Sehstärkenbestimmung (Visusbestimmung) durchgeführt.

Zur Ermittlung des Aufnahmeabstands wird in einer Ausführungsform eine Pupillendistanz des Messsubjekts zugrunde gelegt, die aus früheren Messungen vorliegt oder vor Durchführung der Messaufnahmen gemessen wird, oder es werden Messdaten eines Abstandssensors oder einer Tiefenkamera des mobilen Endgeräts verwendet.

In Ausführungsformen wird oder werden zur Bestimmung des Orientierungswinkels einer Messaufnahme ein Orientierungswinkel der Verbindungslinie der Pupillen des Messsubjekts in der Messaufnahme zugrunde gelegt, und/oder Messdaten von Neigungssensoren, Magnetfeldsensoren und/oder Beschleunigungssensoren des mobilen Endgeräts verwendet.

In Ausführungsformen des Verfahrens wird die Auswertung der Messaufnahmen, insbesondere die Erkennung und Vermessung der Möndchen, und/oder die Refraktionsbestimmung von einer auswertenden Person und/oder unter Verwendung von deterministischen Methoden unterstützt und/oder kontrolliert. Bei der auswertenden Person handelt es sich beispielsweise um einen ausgebildeten Augenoptiker oder Augenarzt. Deterministische Methoden werden beispielsweise zur Erkennung oder Maskierung von Augenpartien oder einzelnen Augen in der Messaufnahme eingesetzt, um dann die weitere Auswertung nur noch anhand der Bilddaten in der erkannten und/der maskierten Augenpartie zu machen. So kann beispielsweise erkannt werden, ob tatsächlich die Bilddaten der Augen des Messsubjekts verarbeitet werden, oder eventuell Bilddaten von einem ebenfalls in der Aufnahme vorhandenen Bild, beispielsweise eines im Hintergrund hängenden Posters mit dem Gesicht einer anderen Person. Im Zweifelsfall kann beispielsweise eine automatische Kontrolle erfolgen, ob die von einem neuronalen Netz ausgewerteten Bildteile mit Bildteilen, die mit deterministischen Methoden automatisch maskiert wurden, übereinstimmen.

In diesem Zusammenhang können als deterministische Methoden beispielsweise die Canny Edge Detection oder die Laplace Edge Detection zur Identifizierung der Randkante der Pupille oder Iris verwendet werden. Zum Auffinden von Kreisen im Bild kann beispielsweise die Hough Circle Transform verwendet werden. Dies ist beispielsweise zur Identifizierung der Pupille oder Iris im Bild nützlich. Ein Rangordnungsfilter (Englisch: "median filter") kann verwendet werden, um Blitzreflektionen oder "Salt and Pepper Noise" zu entfernen oder zu reduzieren.

Gemäß einer Ausführungsform der Erfindung, die die Anwesenheit einer zweiten Person entbehrlich macht, wird bei der Durchführung eines oder mehrerer der Verfahrensschritte a) bis f) eine Sprachausgabe und/oder Sprachsteuerung und/oder eine Fernbedienung über ein entferntes Gerät, welches mit dem mobilen Endgerät kommunikativ verbunden ist, insbesondere eine Smart-Watch oder ein Tablet-Gerät, eingesetzt, wobei in einer Sprachausgabe und/oder einer Ausgabe über das entferne Gerät Anweisungen an das Messsubjekt oder eine Bedienperson gegeben werden, insbesondere in Bezug auf Haltung und Aufnahmeabstand, wobei in einer Sprachsteuerung und/oder einer Steuerung über das entfernte Gerät das Messsubjekt oder eine andere Bedienperson das mobile Endgerät anweist, Testaufnahmen oder Messaufnahmen aufzunehmen und insbesondere auszuwerten. Dies ermöglicht es, das erfindungsgemäße Verfahren mit einem mobilen Endgerät alleine zu Hause oder auch in einem Optikerfachgeschäft durchzuführen.

In Ausführungsformen werden dem Messsubjekt zusätzlich ein oder mehrere subjektive Tests angezeigt, insbesondere ein Astigmatismustest oder ein Rot-Grün-Test. Diese subjektiven Tests können dazu dienen, die Genauigkeit der Messwerte, beispielsweise der Achse, zu erhöhen, oder aber um bei widersprüchlichen Messresultaten aus der objektiven Refraktionsbestimmung im Sinne eines Cross-Checks Klarheit zu schaffen.

Die der Erfindung zugrundeliegende Aufgabe wird auch durch ein Softwareprodukt gelöst, welches ausgelegt ist, auf einem mobilen Endgerät abzulaufen und bei Ablaufen auf dem mobilen Endgerät ein hierüber beschriebenes erfindungsgemäßes Verfahren zur Refraktionsbestimmung auszuführen. Das Softwareprodukt, welches auf einem mobilen Endgerät abläuft, verwirklicht die gleichen Vorteile, Merkmale und Eigenschaften wie das erfindungsgemäße Verfahren. Es kann als Programm, Applikation o. ä. auf dem mobilen Endgerät temporär oder permanent gespeichert werden.

Die der Erfindung zugrunde liegende Aufgabe wird ferner durch ein System mit wenigstens einem zentralen Server und einer Mehrzahl von mobilen Endgeräten, auf denen ein zuvor beschriebenes erfindungsgemäßes Softwareprodukt installiert ist, gelöst, wobei die mobilen Endgeräte mittels des Softwareprodukts eingerichtet sind, Datensätze mit den aufgenommenen Messaufnahmen zusammen mit den endgültig gemessenen Werten für Sphäre, Zylinder und Achse an den wenigstens einen zentralen Server zu übermitteln, wobei die mobilen Endgeräte und/oder der Server eingerichtet ist oder sind, anhand eines Teils oder aller übermittelten Datensätze Machine Learning durchzuführen, wobei insbesondere die mobilen Endgeräte des Systems mittels des Softwareprodukts eingerichtet sind, im Machine Learning gelernte Parameter vom Server abzurufen, insbesondere vor Durchführung einer neuen Messung. Die Übermittlung der Daten von einem mobilen Endgerät an den Server erfolgt vorzugsweise zusammen mit den endgültig gemessenen Werten für Sphäre, Zylinder und Achse, um zum weiteren Trainieren neuronaler Netzwerke für eine oder mehrere der zuvor beschriebenen Analyseaufgaben verwendet zu werden. Wenn die entsprechenden Einstellungen und Parameter, die sich aus dem jeweiligen Training ergeben, vor der Durchführung einer Messung von dem Server abgefragt werden, stehen jeweils die aktuellsten Parameter bereit, um die Messung erfolgreich durchzuführen.

Dies ermöglicht es, in einer Ausführungsform eine zentrale Datenbank zu erstellen, die eine große Anzahl von Referenzdaten zur Verfügung stellt, an denen die zuvor beschriebenen neuronalen Netze trainiert werden können oder andere Formen des Machine Learning ausgeführt werden können. Dies kann ein lokales System sein, wie beispielsweise bei einer Augenarztpraxis oder einem Optikerfachgeschäft, oder ein verteiltes System, wie bei einer Kette von Optikerfachgeschäften oder bei einem ungebundenen Anbieter.

Ein Server-Client-System ermöglicht es auch, die eigentliche Bildauswertung und Berechnung aus dem mobilen Endgerät zu entfernen und in den Server zu verlagern, der gegebenenfalls deutlich höhere Rechenleistungen aufweist als das mobile Endgerät.

Das erfindungsgemäße Verfahren, das erfindungsgemäße Softwareprodukt und das erfindungsgemäße System können auch in den Rahmen eines erweiterten Serviceangebots eingebettet werden, innerhalb dessen der Kunde bzw. der Nutzer nicht nur die Refraktionsbestimmung mittels eines mobilen Endgeräts durchführt oder das Ergebnis über das mobile Endgerät erhält, sondern auch mithilfe des mobilen Endgeräts oder eines Computers weitere Schritte zur Auswahl und Anpassung einer Korrektionsbrille durchführen kann. Dazu gehört beispielsweise die Auswahl der Fassung, wobei die Fassung beispielsweise virtuell auf einem Bild oder einem 3D-Scan des Kopfes des Kunden überlagert angezeigt werden kann, um dem Kunden die Kaufentscheidung zu erleichtern, die Zentrierung der Korrektionsbrille und/oder die virtuelle Anpassung der ausgewählten Brille an die Form des Kopfes und des Gesichts des Kunden. Dies kann im Optikerfachgeschäft erfolgen und/oder beim Einkauf per Fernhandel, d.h. online.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:

Fig. 1    eine schematische Darstellung einer Messanordnung zur exzentrischen Photorefraktion mit einem mobilen Endgerät,

Fig. 2    eine Detaildarstellung einer Messaufnahme mit der Darstellung von Pupille und Möndchen,

Fig. 3    eine schematische Darstellung eines mobilen Endgeräts,

Fig. 4    ein Ablaufdiagramm eines erfindungsgemäßen Verfahrens,

Fig. 5    ein Diagramm mit einer Version einer multimeridionalen Auswertung von Messdaten und

Fig. 6    eine schematische Darstellung eines erfindungsgemäßen Systems.

In den Zeichnungen sind jeweils gleiche oder gleichartige Elemente und/oder Teile mit denselben Bezugsziffern versehen, so dass von einer erneuten Vorstellung jeweils abgesehen wird.

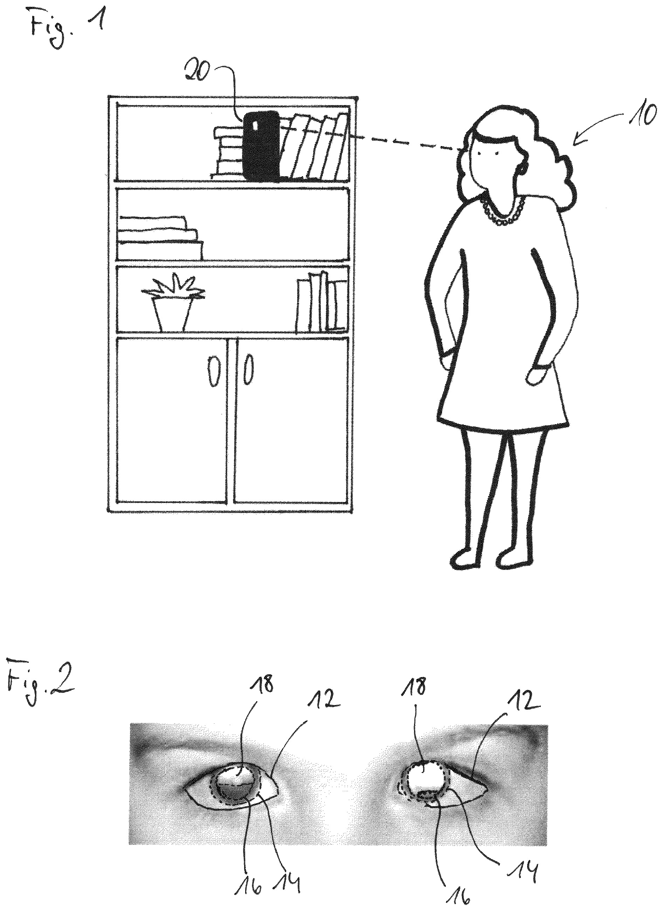

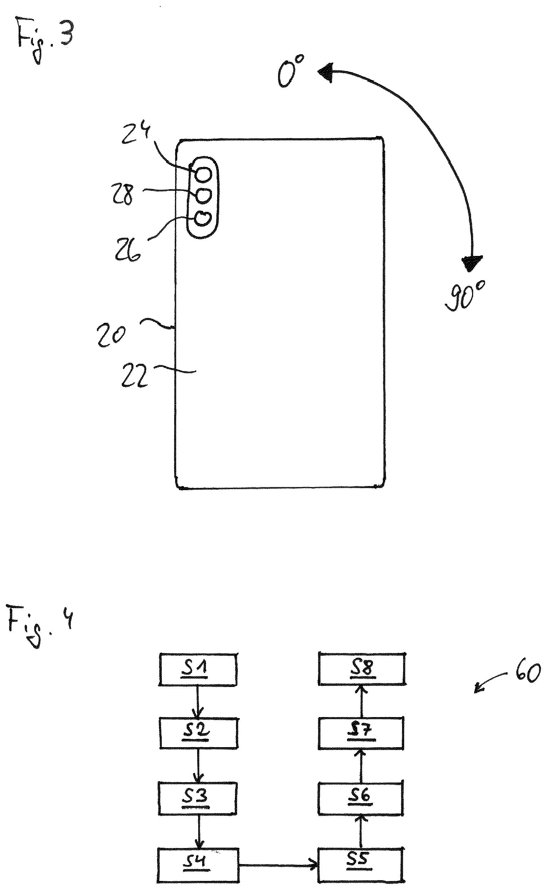

Fig. 1 zeigt eine schematische Darstellung einer Messanordnung zur exzentrischen Photorefraktion mit einem mobilen Endgerät 20. Bei dem mobilen Endgerät 20 handelt es sich um ein Smartphone, welches in einem Bücherregal im Wohnzimmer eines Nutzers 10 so aufgestellt ist, dass die Rückseite mit einer Kamera und einem Blitzlicht, welche in der schematischen Darstellung der Fig. 1 nicht eigenständig dargestellt sind, jedoch wie in Fig. 3 gezeigt ausgebildet sein können, in den Raum gerichtet ist. Die Nutzerin 10 stellt sich zur Durchführung des Verfahrens zur exzentrischen Photorefraktion in den Aufnahmebereich, also das Blickfeld, der Kamera und blickt auf das Blitzlicht des mobilen Endgeräts 20.

Um einen reibungslosen Ablauf des Verfahrens zu ermöglichen, kann das Programm, das auf dem mobilen Endgerät 20 zur Durchführung des Verfahrens abläuft, eine sprachgesteuerte Benutzerschnittstelle aufweisen, und somit der Nutzerin 10 Anweisungen geben, sich beispielsweise zu positionieren oder andere Handlungen auszuführen, oder um Anweisungen von der Nutzerin 10 bitten, beispielsweise, wenn diese sich nach ihrer Ansicht korrekt aufgestellt hat und die Anweisung gibt, nunmehr Testaufnahmen oder Messaufnahmen durchzuführen. Über die Benutzerschnittstelle kann das ablaufende Programm der Nutzerin 10 dann auch rückmelden, ob die Messaufnahme erfolgreich war und ob beispielsweise die Orientierung des mobilen Endgeräts 20 für die nächste Aufnahme geändert werden muss oder eine weitere Messaufnahme unter der gleichen Orientierung stattfinden soll. Anstelle oder zusätzlich zu der sprachgesteuerten Benutzerschnittstelle kann auch eine alternative Art der Fernbedienung über ein Gerät erfolgen, welches dem Benutzer direkt zur manuellen Bedienung zur Verfügung steht, beispielsweise einer Smart-Watch oder einem Tablet, welche bzw. welches mit dem mobilen Endgerät in Kommunikation steht.

In Fig. 2 ist ein Detail einer Messaufnahme mit der Darstellung von Pupille und Möndchen gezeigt, wobei zur Verdeutlichung einige Konturen der interessierenden Details durch Striche oder strichpunktierte Linien hervorgehoben sind. Dargestellt ist die Augenpartie eines Messsubjekts 10 mit den Augen 12, in denen deutlich die im wesentlichen kreisrunden Irides 14 und Pupillen 16 zu sehen sind. Aufgrund der Aufnahme mit Fotolicht oder Blitzlicht unter einer festen Exzentrizität entstehen in den Pupillen auf beiden Seiten Möndchen 18, welche dadurch entstehen, dass aufgrund der Fehlsichtigkeit der beiden Augen ein Teil des in das Auge gelangenden und an der Netzhaut reflektierten Lichts des Blitzlichts nicht zurück zum Blitzlicht gelangen, sondern in die Kamera. Die Größe und Orientierung der Möndchen 18 ist ein Maß für die Fehlsichtigkeit.

Wie in Figur 2 zu sehen, hat das Messsubjekt in den beiden Augen 12 deutlich unterschiedliche Fehlsichtigkeiten. Die Möndchen 18 weisen jeweils einen Helligkeitsverlauf und eine mehr oder weniger gut erkennbare Kante auf, unterhalb derer keine Reflexionen mehr zu erkennen ist. Die Orientierung, der Helligkeitsverlauf, und die allgemeine Form bzw. Positionierung der Kante sind mögliche Auswertungsgrößen für eine Auswertung der Messaufnahmen. Für diese Auswertung existieren unterschiedliche Verfahren, welche zuvor bereits vorgestellt wurden.

Für die Erkennung der Pupillen 16 und Möndchen 18 werden deterministische Verfahren wie Kantendetektion o. ä. Verfahren verwendet, oder aber Machine Learning basierte Verfahren wie beispielsweise hierauf trainierte neuronale Faltungsnetze (CNN).

Die Rückseite 22 eines geeigneten mobilen Endgeräts 20 ist in Fig. 3 schematisch dargestellt. Sie weist einen Block in der linken oberen Ecke mit zwei Kameras 24, 26 und einem Fotolicht 28 auf. Bei den Kameras 24, 26 kann es sich beispielsweise um Kameras für den Weitwinkelbereich und den Telebereich handeln, wobei für die Testaufnahmen beispielsweise beide Kameras 24, 26 verwendet werden können, um die Ausrichtung bzw. Positionierung des Nutzers 10 zu überprüfen. Für die Messaufnahmen wird jeweils das Fotolicht 28 eingeschaltet, um den Effekt der Möndchen 18 in den Pupillen 16 der Augen 12 des Nutzers 10 zu erzeugen.

Ebenfalls ist in Figur 3 dargestellt, dass durch eine Drehung des mobilen Endgeräts 20 um eine Achse, die parallel zur optischen Achse der Kameras 24, 26 ist, die Orientierung des Blitzlichts bzw. Fotolichts 28 relativ zu den Kameras 24, 26 geändert werden kann, um eine bimeridionale oder multimeridionale Auswertung zu ermöglichen. Die gezeigten Winkel 0° und 90° entsprechen den Winkeln des Hochformats und des Querformats für eine bimeridionale Auswertung. Für eine multimeridionale Auswertung können zusätzlich andere Winkel, beispielsweise im 30°-Abstand verwendet werden. Auch die Verwendung von 0° oder 90° sind für eine multimeridionale Auswertung nicht zwingend. Das gleiche gilt für die bimeridionale Auswertung. Wesentlich ist hierfür, dass zwei unterschiedliche Winkel verwendet werden, wobei die besten Ergebnisse im Allgemeinen erzielt werden, wenn diese beiden Winkel senkrecht auf einander stehen.

Fig. 4 zeigt ein Ablaufdiagramm eines Ausführungsbeispiels eines erfindungsgemäßen Verfahrens 60. Verfahrensschritt S1 besteht in der Vorbereitung der Messung. Hier wird sichergestellt, dass der Nutzer 10 auf den Blitz schaut und der Raum gegebenenfalls abgedunkelt wird, um sonstige Lichtreflexe im Auge zu vermeiden. Der Nutzer 10 beziehungsweise das Messsubjekt muss versuchen, auf das Blitzlicht zu fokussieren, darf dieses jedoch nicht scharf sehen. Die Pupillendistanz PD muss bekannt sein oder an dieser Stelle vermessen werden.

Im Verfahrensschritt S2 werden die Kameraeinstellungen festgelegt. Beispielhaft für die in diesem Schritt vorzunehmenden Einstellungen wird gegebenenfalls ein geeignetes Objektiv ausgewählt und für die Auswertung gemerkt. Die zuständige App oder das zuständige Programm auf dem mobilen Endgerät 20 wird ausgewählt und gegebenenfalls ein Blitzgerät oder Blitzlicht eingeschaltet. Dabei kann ausgewählt werden, dass die Messaufnahmen auch als unkomprimierte Rohdaten gespeichert werden, um die größtmögliche Bildinformationen für die spätere Auswertung zur Verfügung zu haben und Artefakte bei einer internen JPEG-Komprimierung zu vermeiden, welche die Auswertung stören könnten. Ferner wird der Fokus gegebenenfalls manuell auf die Augenpartie des Nutzers gesetzt. Diese Einstellungen können manuell oder gegebenenfalls von dem Programm bzw. der Applikation vorgenommen werden und sind nicht als abschließend zu verstehen.

Im nachfolgenden Verfahrensschritt S3 wird eine Abstandsbestimmung bzw. -optimierung zwischen der Kamera bzw. dem mobilen Endgerät 20 und dem Nutzer 10 durchgeführt. Durch Testfotos ist das Messsubjekt bzw. Nutzer 10 so vor dem mobilen Endgerät 20 zu positionieren, dass das Möndchen 18 in den Testfotos idealerweise etwa die Hälfte der Pupille 16 füllt. Dies hängt von dem individuellen Sehfehler des Nutzers 10 ab, wobei die Entfernung auch nicht zu groß sein darf, damit die Pupillen 16 eine ausreichende Anzahl an Pixeln in den Messaufnahmen bzw. Testaufnahmen überdecken.

Nachdem auf diese Weise der Nutzer platziert worden ist, erfolgen im nachfolgenden Verfahrensschritt S4 Messaufnahmen unter verschiedenen Winkeln. Dabei muss sichergestellt werden, dass für das verwendete Objektiv der Kamera die richtigen Winkel gewählt werden und vorzugsweise mehrere Fotos pro Einstellung aufgenommen werden. Für eine bimeridionale Auswertung genügen Aufnahmen unter 0° und 90°, also hochkant und flach seitlich, während eine geeignete Winkelfolge für eine multimeridionale Auswertung beispielsweise eine Abfolge von Winkeln in 30°-Schritten ist, um die das mobile Endgerät 20 beispielsweise aus der Horizontalen mit dem oberen Bildschirmrand zunächst beispielsweise nach links in die entgegengesetzte Horizontale mit dem oberen Bildschirmrand nach rechts gebracht wird, oder umgekehrt.

Im Verfahrensschritt S5 wird die Pupillendistanz (PD) in Pixeln vermessen. Dies kann beispielsweise anhand der Lichtreflexe in den Pupillen erfolgen, wobei die Pupillendistanz zunächst in Pixeln gemessen wird. Die genaue Entfernung wird anhand der vorhandenen Pupillendistanz in Metern und dem Maßstab von Meter pro Pixel umgerechnet, welcher eine Eigenschaft des Objektivs ist und bei bekanntem Abstand zum Nutzer eine einfache Umrechnung erlaubt. Hierbei wird je nach Objektiv der richtige Umrechnungsfaktor verwendet.

Verfahrensschritt S6 beinhaltet eine Winkelbestimmung, wobei der Winkel der Kamera 24, 26 und somit der Winkel der Verbindungslinie zwischen der verwendeten Kamera 24, 26 und dem verwendeten Fotolicht 28 relativ zur Verbindungslinie zwischen den Pupillen 16 des Nutzers 10 berechnet wird. Dieser Winkel entspricht der Winkellage des für die Messaufnahme gültigen Meridians.

Der Verfahrensschritt S7 besteht aus der Vermessung des Möndchens 18 in den Pupillen 16 des Nutzers 10. Dabei kann entweder der helle Anteil oder der dunkle Anteil der Pupillen 16 vermessen werden. Es ist darauf zu achten, dass das Möndchen 18 im richtigen Bereich der Pupille 16 liegt. Außerdem sollte das linke und das rechte Auge des Nutzers 10 nicht miteinander vertauscht werden. Aus den einzelnen Messaufnahmen mit den vermessenen Möndchen 18 werden die für den jeweiligen Meridian gültigen Dioptrienwerte ermittelt. Verfahren zur Vermessung des Möndchens 18 und zur Ermittlung der Dioptrienwerte hieraus sind weiter oben bereits vorgestellt worden. Das Verfahren 60 mündet in den Verfahrensschritt S8, in welchem eine Auswertung der Abhängigkeit der ermittelten Dioptrienwerte von den jeweiligen Meridianen, d. h. den Winkellagen, erfolgt. Auch hierfür sind weiter oben verschiedene Verfahren vorgestellt worden. Ein Beispiel ist eine Anpassung einer Fitfunktion mithilfe einer nichtlinearen Regression. Diese Regression liefert die Werte für Sphäre, Zylinder und Achse in der jeweils gewählten Notation.

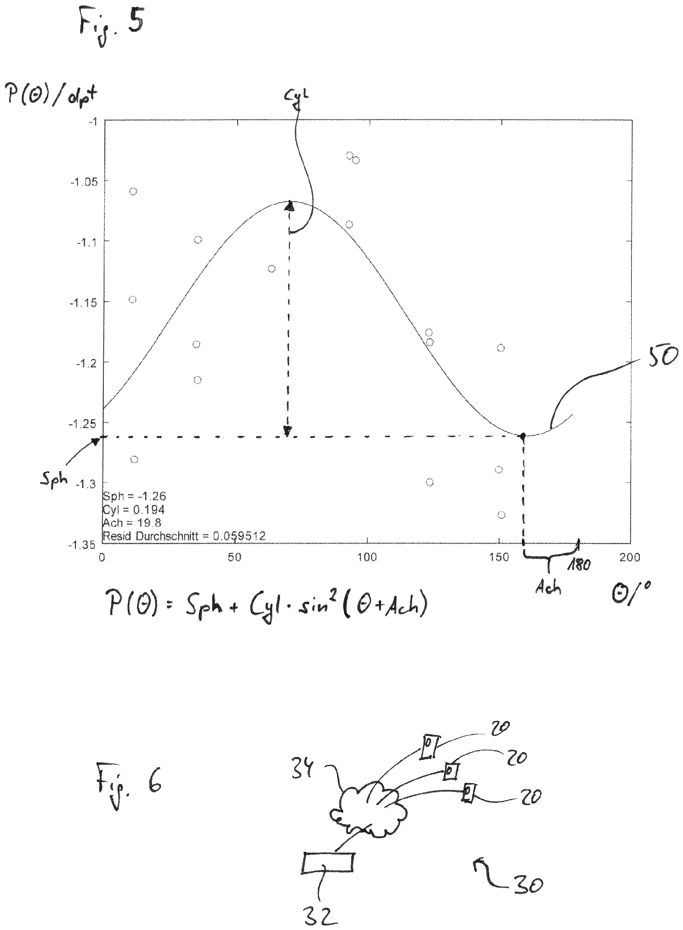

Fig. 5 zeigt ein Diagramm mit einer Version einer multimeridionalen Auswertung von Messdaten. Die Punkte repräsentieren einzelne Dioptrienwerte, die aus verschiedenen Messaufnahmen bei verschiedenen Meridian genommen worden sind. So wurden jeweils drei Messaufnahmen bei Meridianen bei ca. 10°, 35°, 60° (hier nur eine Aufnahme), 90°, 125° und 150° aufgenommen und ausgewertet. Die Dioptrienwerte weisen eine gewisse Streuung auf, bewegen sich allerdings in einem vergleichsweise engen Rahmen von bis zu ±0,1 dpt, was bereits eine sehr gute Genauigkeit darstellt. Deutlich zu erkennen ist, dass die Werte im mittleren Winkelbereich um die 100° am stärksten positiv sind. Die in Figur 5 beispielhaft verwendete Fitfunktion

$$P(\theta) = Sph + Cyl \cdot \sin^2(\theta + Ach)$$

ergibt für die gezeigten Messwerte einen Fit mit den Werten

*Sph* = -1,26 dpt, *Cyl* = 0,194 dpt und *Achse* = 19,8°.

Der residuale Durchschnitt ist ca. 0,06 dpt, was bedeutet, dass die Messwerte im Durchschnitt nicht weiter als 0,06 Dioptrien von der Fitkurve 50 entfernt sind. Ebenfalls ist in Figur 5 markiert, dass der Wert der Sphäre *Sph* in der gewählten Notation den negativeren der Extremwerte der Fitfunktion 50 bedeutet und Zylinder *Cyl* die Amplitude zwischen dem Minimum und dem Maximum. Die Achse *Ach* bezeichnet die Winkelverschiebung zwischen dem Minimum und dem 0°-Punkt im Minimum der Fitfunktion 50, also dem negativsten Wert. Das Ergebnis sind die Werte, die im Brillenpass eingetragen werden, wobei diese Werte als Ausgangspunkt für eine subjektive Augenglasbestimmung dienen können.

Fig. 6 stellt ein erfindungsgemäßes System 30 schematisch dar. Das System verfügt über einen zentralen Server 32, der über das Internet 34 mit einer Mehrzahl von mobilen Endgeräten 20 verbunden ist, welche jeweils mit einer erfindungsgemäßen Software bespielt sind. Der zentrale Server 32 kann Messaufnahmen der mobilen Endgeräte 20 empfangen und auf deren Grundlage neuronale Netze beispielsweise anpassen und trainieren, oder andere Arten von Machine Learning ausführen, wobei die Endgeräte 20 ihre Parameter für die eigene Auswertung oder die Ergebnisse einer am zentralen Server 32 ausgeführten Auswertung empfangen. Das System 30 ist geeignet, eine große Datenbasis für das erfindungsgemäße Verfahren zusammenzustellen und somit die Auswertung von Messaufnahmen laufend zu optimieren.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

Bezugszeichenliste

| | |
|---|---|
| 10 | Nutzer |
| 12 | Auge |
| 14 | Iris |
| 16 | Pupille |
| 18 | Möndchen |
| 20 | mobiles Endgerät |
| 22 | Rückseite |
| 24 | Kamera |
| 26 | Kamera |
| 28 | Fotolicht |
| 30 | System |
| 32 | Server |
| 34 | Internet |
| 50 | Fitkurve |
| 60 | Verfahren |
| S1 | Vorbereitung |
| S2 | Kameraeinstellung |
| S3 | Abstandsbestimmung |
| S4 | Winkelaufnahmen |
| S5 | Vermessen der Pupillendistanz |
| S6 | Winkelbestimmung |
| S7 | Vermessen der Möndchen |
| S8 | Auswertung |

**Patentansprüche**

1. Verfahren zur Refraktionsbestimmung an einem Messsubjekt, (10) dessen Augen einer exzentrischen Photorefraktion zu unterziehen sind, auf einem mobilen Endgerät (20), welches eine Kamera (24, 26) mit einer Linse und eine schaltbare Lichtquelle (28) umfasst, die mit einem festen Abstand neben der Linse angeordnet ist, mit den folgenden Verfahrensschritten:

a) Positionieren des mobilen Endgeräts (20) in einem initialen Aufnahmeabstand A vor dem Messsubjekt (10), so dass die Kamera (24, 26) und die Lichtquelle (28) auf das Gesicht des Messsubjekts (10) gerichtet sind,
b) Aufnehmen von Testbildern des Messsubjekts (10) ohne und/oder mit zugeschaltetem Licht aus der Lichtquelle (28) und Optimieren des Aufnahmeabstands A in Abhängigkeit von der Brechkraft des Auges oder der Augen des Messsubjekts (10) sowie Optimieren von Aufnahmeparametern, von Lichtbedingungen und/oder der Ausrichtung des mobilen Endgeräts (20) zum Messsubjekt (10) anhand der Testbilder, bis eine zur exzentrischen Photorefraktion geeignete Einstellung gefunden ist,
c) Aufnehmen einer Mehrzahl von Messaufnahmen des Messsubjekts (10), wobei das mobile Endgerät (20) durch Rotation um eine Achse, die parallel zur optischen Achse der Linse verläuft, nacheinander in zwei oder mehr Winkelorientierungen gebracht wird und in jeder Winkelorientierung eine oder mehrere Messaufnahmen aufgenommen werden,
d) Auswerten der Mehrzahl von Messaufnahmen im Zuge einer Refraktionsauswertung, wobei Werte für die winkelabhängige Brechkraft eines oder beider Augen (12) ermittelt werden, insbesondere Werte für *Sph, Cyl* und *Achse,*
e) Ausgeben und/oder Speichern der Messergebnisse, insbesondere der Werte für *Sph, Cyl* und *Achse* eines oder beider Augen (12) des Messsubjekts (10),

wobei bei der Auswertung der Testbilder, der Messaufnahmen und/oder bei der Refraktionsauswertung Machine Learning, insbesondere neuronale Netze, in dem mobilen Endgerät oder in einem mit dem mobilen Endgerät datenverbundenen Server (32) eingesetzt wird oder werden.

2. Verfahren nach Anspruch 1, **dadurch gekennzeichnet, dass** an der Mehrzahl von Testbildern und/oder Messaufnahmen eine bimeridionale oder multimeridionale Refraktionsauswertung ausgeführt wird, wobei an der Mehrzahl von Testbildern und/oder Messaufnahmen jeweils eine Bildanalyse durchgeführt wird, wobei in den Messaufnahmen das Vorhandensein, Größe, Orientierung, Ort, Form und/oder Intensitätsverlauf von reflektiertem Licht, welches in den Messaufnahmen als Möndchen (18) in den Pupillen (16) des Messsubjekts (10) erscheint, sowie der Aufnahmeabstand A ermittelt werden, wobei insbesondere für die bimeridionale Auswertung Messaufnahmen in zwei zueinander senkrecht stehenden Winkelorientierungen aufgenommen werden, insbesondere im Hoch- und Querformat, und/oder für die multimeridionale Auswertung Messaufnahmen in wenigstens drei, vorzugsweise vier, fünf oder mehr, verschiedenen Winkelorientierungen aufgenommen werden, insbesondere in Winkelabständen von 30°.

3. Verfahren nach Anspruch 1 oder 2, **dadurch gekennzeichnet, dass** ein Aufnahmeabstand gewählt wird, der etwas, insbesondere bis zu 40%, insbesondere bis zu 20%, größer als der Fernpunkt wenigstens eines Auges (12) des Messsubjekts (10) ist.

4. Verfahren nach einem der Ansprüche 1 bis 3, **dadurch gekennzeichnet, dass** im Verfahrensschritt b) oder c) zu jeder Messaufnahme geprüft wird, ob diese verwertbar ist, und eine Änderung der Winkelorientierung des Endgeräts (20) dann veranlasst wird, wenn in der bisherigen Winkelorientierung eine festgelegte oder ausreichende Anzahl verwertbarer Messaufnahmen aufgenommen worden ist, wobei insbesondere die Bildanalyse einer Messaufnahme unmittelbar nach dem Aufnehmen der Messaufnahme im Verfahrensschritt c) erfolgt.

5. Verfahren nach einem der Ansprüche 1 bis 4, **dadurch gekennzeichnet, dass** in den Messaufnahmen das Gesicht des Messsubjekts (10), die Augen (12), die Iris (14) und/oder die Pupille(n) (16) erkannt wird oder werden und ihre Größe, insbesondere in Pixeln, bestimmt werden, sowie das oder die Möndchen (18) innerhalb der erkannten Iris oder Irides (14) erkannt wird oder werden.

6. Verfahren nach Anspruch 5, **dadurch gekennzeichnet, dass** bei der Erkennung des Gesichts, der Augen (12), der Iris (14), der Pupille (16) und/oder des Möndchens (18) ein oder mehrere neuronale Netzwerke eingesetzt werden, das oder die zuvor für die entsprechende Erkennung trainiert worden war oder waren, wobei insbesondere bei der Bestimmung von Größe, Lage und/oder Helligkeitsverlauf des Möndchens (18) oder der Möndchen (18) eine durch Machine Learning unterstützte Superresolution verwendet wird, die insbesondere unter Verwendung von neuronalen Netzen durchgeführt wird, die anhand von Bildern in hoher und niedriger Auflösung trainiert worden sind, insbesondere dahingehend, die Kante eines Möndchens in der Pupille zu erkennen und zu verdeutlichen.

7. Verfahren nach einem der Ansprüche 1 bis 6, **dadurch gekennzeichnet, dass** ein System künstlicher Intelligenz, insbesondere Machine Learning oder ein neuronales Netzwerk, eingesetzt wird, um ausgehend von einem oder mehreren Messaufnahmen mit Möndchen (18) und den zugehörigen Messparametern Messabstand A und Exzentrizität e der Lichtquelle Messwerte für *Sph, Cyl* und *Achse* zu ermitteln, wobei das System künstlicher Intelligenz zuvor mit Messaufnahmen mit Möndchen (18) von Referenzsubjekten mit bekannten Werten von *Sph, Cyl* und *Achse* trainiert worden war.

8. Verfahren nach einem der Ansprüche 1 bis 7, **dadurch gekennzeichnet, dass** Güte und/oder Verlässlichkeit der Messergebnisse berechnet wird oder werden und bei nicht ausreichender Güte und/oder Verlässlichkeit das Messsubjekt (10) aufgefordert wird, einzelne oder alle Schritte der Messung zu wiederholen oder eine Refraktionsbestimmung beim Augenoptiker durchführen zu lassen.

9. Verfahren nach einem der Ansprüche 1 bis 8, **dadurch gekennzeichnet, dass** zur Ermittlung des Aufnahmeabstands A eine Pupillendistanz (PD) des Messsubjekts (10) zugrunde gelegt wird, die aus früheren Messungen vorliegt oder vor Durchführung der Messaufnahmen gemessen wird, oder Messdaten eines Abstandssensors oder einer Tiefenkamera des mobilen Endgeräts (20) verwendet werden.

10. Verfahren nach einem der Ansprüche 1 bis 9, **dadurch gekennzeichnet, dass** zur Bestimmung des Orientierungswinkels einer Messaufnahme ein Orientierungswinkel der Verbindungslinie der Pupillen (16) des Messsubjekts (10) in der Messaufnahme zugrunde gelegt wird, und/oder Messdaten von Neigungssensoren, Magnetfeldsensoren und/oder Beschleunigungssensoren des mobilen Endgeräts (20) verwendet werden.

11. Verfahren nach einem der Ansprüche 1 bis 10, **dadurch gekennzeichnet, dass** die Auswertung der Messaufnahmen, insbesondere die Erkennung und Vermessung der Möndchen (18), und/ oder die Refraktionsbestimmung von einer auswertenden Person und/oder unter Verwendung von deterministischen Methoden unterstützt und/oder kontrolliert wird.

12. Verfahren nach einem der Ansprüche 1 bis 11, **dadurch gekennzeichnet, dass** bei der Durchführung eines oder mehrerer der Verfahrensschritte a) bis f) eine Sprachausgabe und/oder Sprachsteuerung und/oder eine Fernbedienung über ein entferntes Gerät, welches mit dem mobilen Endgerät kommunikativ verbunden ist, insbesondere eine Smart-Watch oder ein Tablet-Gerät, eingesetzt wird, wobei in einer Sprachausgabe und/oder einer Ausgabe über das entferne Gerät Anweisungen an das Messsubjekt (10) oder eine Bedienperson gegeben werden, insbesondere in Bezug auf Haltung und Aufnahmeabstand, wobei in einer Sprachsteuerung und/oder einer Steuerung über das entferne Gerät das Messsubjekt (10) oder eine andere Bedienperson das mobile Endgerät (20) anweist, Testaufnahmen oder Messaufnahmen aufzunehmen und insbesondere auszuwerten.

13. Verfahren nach einem der Ansprüche 1 bis 12, **dadurch gekennzeichnet, dass** zusätzlich eine Sehstärkenbestimmung (Visusbestimmung) durchgeführt wird und/oder dem Messsubjekt (10) zusätzlich ein oder mehrere subjektive Tests angezeigt werden, insbesondere ein Astigmatismustest oder ein Rot-Grün-Test.

14. Softwareprodukt, welches ausgelegt ist, auf einem mobilen Endgerät (20) abzulaufen und bei Ablaufen auf dem mobilen Endgerät (20) ein Verfahren zur Refraktionsbestimmung gemäß einem der Ansprüche 1 bis 13 auszuführen.

15. System (30) mit wenigstens einem zentralen Server (32) und einer Mehrzahl von mobilen Endgeräten (20), auf denen ein Softwareprodukt gemäß Anspruch 14 installiert ist, wobei die mobilen Endgeräte (20) mittels des Softwareprodukts eingerichtet sind, Datensätze mit den aufgenommenen Messaufnahmen, insbesondere zusammen mit den endgültig gemessenen Werten für *Sph, Cyl* und *Achse,* an den wenigstens einen zentralen Server (32) zu übermitteln, wobei der Server (32) eingerichtet ist, anhand eines Teils oder aller übermittelten Datensätze Machine Learning durchzuführen, wobei insbesondere die mobilen Endgeräte (20) des Systems (30) mittels des Softwareprodukts eingerichtet sind, im Machine Learning gelernte Parameter vom Server (32) abzurufen, insbesondere vor Durchführung einer neuen Photorefraktionsbestimmung.

Fig. 1

Fig. 2

Fig. 3

Fig. 4

# Fig. 5

$P(\Theta)/dpt$

Sph = -1.26
Cyl = 0.194
Ach = 19.8
Resid Durchschnitt = 0.059512

$$P(\Theta) = Sph + Cyl \cdot \sin^2(\Theta + Ach)$$

# Fig. 6

**Europäisches Patentamt**
**European Patent Office**
**Office européen des brevets**

## EUROPÄISCHER RECHERCHENBERICHT

Nummer der Anmeldung

EP 20 15 4521

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | DE 10 2016 112023 A1 (ZEISS CARL AG [DE]; ZEISS CARL VISION INT GMBH [DE]) 4. Januar 2018 (2018-01-04) | 1-5,8-15 | INV. A61B3/00 A61B3/103 A61B3/14 |
| A | * Absatz [0072] - Absatz [0073] * * Absatz [0076] * * Absatz [0079] * * Absatz [0100] * * Absatz [0103] * * Absatz [0107] - Absatz [0109] * * Absatz [0112] - Absatz [0116] * * Absatz [0118] * * Abbildungen 1,2-4,7,9 * | 6,7 | |
| X | WO 2013/036629 A2 (ICHECK HEALTH CONNECTION INC [US]; HUANG DAVID [US] ET AL.) 14. März 2013 (2013-03-14) | 1-5,8-15 | |
| A | * Seite 7, Zeile 31 - Seite 8, Zeile 23 * * Seite 9, Zeile 22 - Seite 10, Zeile 20 * * Abbildungen 1,2A-6D, 15, 16 * | 6,7 | |
| A | EP 3 430 974 A1 (SONY CORP [JP]) 23. Januar 2019 (2019-01-23) * Absatz [0018] * * Absatz [0036] * * Abbildungen 3,4 * | 1-15 | RECHERCHIERTE SACHGEBIETE (IPC) A61B |

Der vorliegende Recherchenbericht wurde für alle Patentansprüche erstellt

| Recherchenort | Abschlußdatum der Recherche | Prüfer |
|---|---|---|
| München | 20. Mai 2020 | Horváth, Lâszlô |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 20 15 4521

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-05-2020

| Im Recherchenbericht angeführtes Patentdokument | Datum der Veröffentlichung | Mitglied(er) der Patentfamilie | Datum der Veröffentlichung |
|---|---|---|---|
| DE 102016112023 A1 | 04-01-2018 | BR 112018077475 A2 | 02-04-2019 |
| | | CN 109688899 A | 26-04-2019 |
| | | CN 110420008 A | 08-11-2019 |
| | | DE 102016112023 A1 | 04-01-2018 |
| | | EP 3478153 A2 | 08-05-2019 |
| | | EP 3569138 A1 | 20-11-2019 |
| | | KR 20190018016 A | 20-02-2019 |
| | | US 2019167093 A1 | 06-06-2019 |
| | | US 2019350454 A1 | 21-11-2019 |
| | | WO 2018002332 A2 | 04-01-2018 |
| WO 2013036629 A2 | 14-03-2013 | AU 2012304575 A1 | 24-04-2014 |
| | | CA 2848206 A1 | 14-03-2013 |
| | | EP 2753228 A2 | 16-07-2014 |
| | | JP 6072798 B2 | 01-02-2017 |
| | | JP 2014526312 A | 06-10-2014 |
| | | KR 20140103900 A | 27-08-2014 |
| | | US 2013235346 A1 | 12-09-2013 |
| | | WO 2013036629 A2 | 14-03-2013 |
| EP 3430974 A1 | 23-01-2019 | CN 109285602 A | 29-01-2019 |
| | | EP 3430974 A1 | 23-01-2019 |
| | | US 2019021588 A1 | 24-01-2019 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 9380938 B2
- US 9877649 B2
- DE 102016112023 A1


**In der Beschreibung aufgeführte Nicht-Patentliteratur**

- **T.D. COLE.** Multimeridian photorefraction: A Technique for the detection of visual defects in infants and preverbal children. *Johns Hopkins APL Technical Digest,* 1991, vol. 12 (2
- **A. ROORDA ; M. C. W. CAMPBELL.** Geometrical theory to predict eccentric photorefraction intensity profiles in the human eye. *Journal ofthe Optical Society of America A,* 1995, vol. 12 (8
- **W. WESEMANN ; A. M. NORCIA ; D. ALLEN.** Theory of eccentric photorefraction (photoretinoscopy): astigmatic eyes. *Journal of the Optical Society of America A,* 1991, vol. 8 (12
- **KUSEL et al.** Light-intensity distribution in eccentric photorefraction crescents. *J. Opt. Soc. Am. A,* 1998, vol. 15 (6
- **ROORDA et al.** Geometrical theory to predict eccentric photorefraction intensity profiles in the human eye. *J. Opt. Soc. Am. A,* 1995, vol. 12 (8
- **COSTA et al.** Photorefraction images analysis through neural networks. *Optics,* 2010